# FASCICULE DE BREVET EUROPEEN

(11) **EP 1 427 350 B1**
(45) Date de publication et mention de la délivrance du brevet: **13.12.2006**
(21) Numéro de dépôt: 02774921.7
(22) Date de dépôt: 20.09.2002
(51) Int. Cl.: A61F 2/00

(54) **Implant complet et universel pour la réparation des hernies par voie antérieure**
Universelles und komplettes Implantat zum äusseren Zugriff für Herniereparatur
Complete and universal implant for front path hernia repair

(30) Priorité: 21.09.2001 FR 0112228
(43) Date de publication de la demande: 16.06.2004
(73) Titulaire: SOFRADIM PRODUCTION, 01600 Trévoux (FR)
(72) Inventeur: NEGRO, Paolo, I-00187 Roma (IT)
(74) Mandataire: Brédeville, Odile Marie
(86) Numéro de dépôt international: PCT/FR2002/003226
(87) Numéro de publication internationale: WO 2003/026530

(56) Documents cités:
- WO-A-02/07648
- DE-A- 10 019 604
- DE-A- 19 832 634
- US-A- 4 854 316
- US-B2- 6 436 030

## Description

La présente invention concerne un implant de traitement de hernies, en particulier de hernies inguinales, fémorales ou crurales, par voie antérieure. Elle concerne également un procédé de fabrication de cet implant.

Il est connu de traiter une hernie inguinale, fémorale ou crurale en utilisant conjointement un implant plat sous forme de nappe, couramment dénommé "patch" et un implant tridimensionnel formant obturateur, couramment dénommé "plug". Le "patch" permet le renforcement de la paroi postérieure du canal inguinal et contribue à éviter le risque de récidive. Le "plug" est introduit profondément dans l'évidement ("defect") laissé par la réduction de la hernie, qu'il permet d'obturer. Le document EP0898944 décrit un implant complet et universel pour la réparation des hernies par voie antérieure correspondant au préambule de la revendication 1.

Il est également connu, par le document WO 97/35533 de réaliser un implant à deux couches faites de matériaux respectivement différents, dont l'une favorise l'adhésion des tissus et l'autre empêche une telle adhésion. Le pliage adéquat de l'implant permet de jouer avec les propriétés respectives de ces couches. Selon une forme de réalisation décrite par ce document, l'implant comprend une excroissance expansible de forme tronconique, aménagée dans la couche adhérente.

Cette excroissance souple et tronconique ne semble pas être à même de parfaitement s'adapter à l'évidement laissé par la réduction de la hernie, ni à même de parfaitement s'opposer à la pression que le "sac" herniaire va exercer contre elle une fois l'implant mis en place. Il en résulte un risque de récidive non négligeable, notamment à la suite d'une éversion de l'excroissance sous ladite pression.

Certes, ce document antérieur expose une possibilité de remplissage de la cavité délimitée par cette excroissance et par la couche non adhérente, au moyen d'un gaz inerte ou d'un autre matériau inerte approprié, ce qui apparaît susceptible d'apporter une certaine rigidité à l'excroissance.

Cette technique de remplissage est toutefois estimée comme impliquant un certain nombre d'inconvénients sérieux de mise en oeuvre, et comme non souhaitable d'une manière générale. De plus, la rigidification de l'excroissance ainsi réalisée est de toute façon temporaire puisqu'il est indiqué par le document antérieur que cette excroissance, lorsqu'elle est ainsi remplie, peut se comprimer au cours du temps du fait de la contraction des tissus environnant l'évidement herniaire.

L'invention vise à remédier à l'ensemble de ces inconvénients

L'implant correspond à la revendication 1; l'implant étant réalisé à partir d'un tricot formé de deux nappes tricotés selon les barèmes suivants : nappe 1 : 32/01/12/43; nappe 2 : 32/01/12/43. Ces armures forment des mailles indémaillables donnant au tricot une semi-rigidité avec néanmoins une possibilité de déformation.

La forme de l'excroissance confère une relative rigidité intrinsèque, lui permettant de parfaitement s'opposer à la pression que le "sac" herniaire exerce contre ledit fond une fois l'implant mis en place. Une prévention efficace du risque d'éversion est ainsi obtenue.

Cette même forme permet à l'excroissance de s'adapter relativement précisément à la forme de l'évidement herniaire, de sorte que ladite paroi périphérique vient au contact des tissus environnants, qui soutiennent cette paroi et contribuent ainsi à la rigidité de l'excroissance.

Après mise en place, l'excroissance peut être fixée à ces tissus environnants au moyen d'une ou plusieurs sutures ; la partie plane peut aussi être fixée aux tissus environnants, notamment aux ligaments inguinaux ou au plancher inguinal, par une ou plusieurs sutures.

L'excroissance est aménagée sensiblement au milieu de la largeur de la partie plane et sensiblement au tiers de la longueur de cette partie plane.

La partie plane peut être découpée en fonction de la taille de la dissection réalisée, pour épouser parfaitement la paroi postérieure du canal inguinal. Ce découpage s'effectue sans effilochage et avec une émission d'une quantité minimale de particules, grâce à l'armure indémaillable constituant le tricot.

Dans une forme d'exécution de l'invention, l'excroissance a un diamètre de l'ordre de 20 millimètres et une hauteur non supérieure à ce diamètre.

La partie plane peut être rectangulaire, avec des angles droits ou arrondis. Elle peut avoir une largeur de l'ordre de 60 millimètres et a une longueur de l'ordre de 120 millimètres.

En raison de sa configuration et de ses dimensions, cet implant convient à tous types de réparation herniaire, indirecte, directe ou crurale.

Dans une forme d'exécution, la face de la partie plane destinée à venir en contact avec les structures anatomiques postérieures présente une rugosité résultant de la texture et/ou de l'armure du tricot composant cette partie plane.

La partie plane et/ou l'excroissance peuvent être imprégnées de substances bactéricides et/ou anti-inflammatoires et/ou analgésiques.

La partie plane peut également présenter localement une fente pour le passage du cordon spermatique.

L'invention concerne également un procédé d'obtention de l'implant décrit ci-dessus.

Ce procédé comprend les étapes consistant à :
a) réaliser un tricot en matériau biocompatible thermoformable, en monofilaments, et
b) réaliser un emboutissage du tricot selon la forme de l'excroissance à aménager et un chauffage de ce tricot, cet emboutissage et ce chauffage pouvant être réalisés simultanément ou successivement.

Le tricot réalisé à l'étape a) présente avantageusement des dimensions telles qu'après formage de l'excroissance, il permet de former également ladite partie plane, telle quelle ou après découpe adéquate.

Le procédé peut comprendre, entre les étapes a) et b) citées ci-dessus, les étapes consistant à :
- placer le tricot entre deux plaques de matériau conducteur de la chaleur, dont une première comprend un trou de diamètre supérieur au diamètre externe de l'excroissance à réaliser et dont la deuxième comprend un plot de diamètre légèrement inférieur au diamètre interne de l'excroissance à réaliser ;
- rapprocher ces deux plaques de telle sorte que le plot de la deuxième plaque soit engagé dans le trou de la première plaque, réalisant ainsi ledit emboutissage, et
- maintenir ces plaques ainsi rapprochées et les chauffer pendant la durée nécessaire à la formation de l'excroissance.

En variante, ladite deuxième plaque peut comprendre, en lieu et place dudit plot, un trou de diamètre au moins égal au diamètre interne de l'excroissance à réaliser, et un mandrin chauffant est engagé au travers des trous des deux plaques pour aménager l'excroissance.

Le rapprochement des plaques est tel que le tricot peut glisser entre les plaques lors de l'aménagement de l'excroissance au moyen dudit plot ou dudit mandrin.

Ce glissement permet de conserver une densité de matériau restant importante au niveau de l'excroissance.

L'invention sera bien comprise à l'aide de la description qui suit de deux formes d'exécution de l'implant, cette description étant faite en référence au dessin schématique annexé. Dans ce dessin,
les figures 1 et 2 sont des vues respectivement en perspective et en coupe longitudinale de l'implant selon une première forme d'exécution ;
la figure 3 est une vue en coupe longitudinale de cet implant lorsqu'il est mis en place dans un évidement herniaire ;
les figures 4, 5 et 6 sont des vues partielles de cet implant, montrant les conditions de mise en place de l'implant pour la réparation, respectivement, d'une hernie gauche indirecte, d'une hernie gauche directe, et d'une hernie crurale droite ;

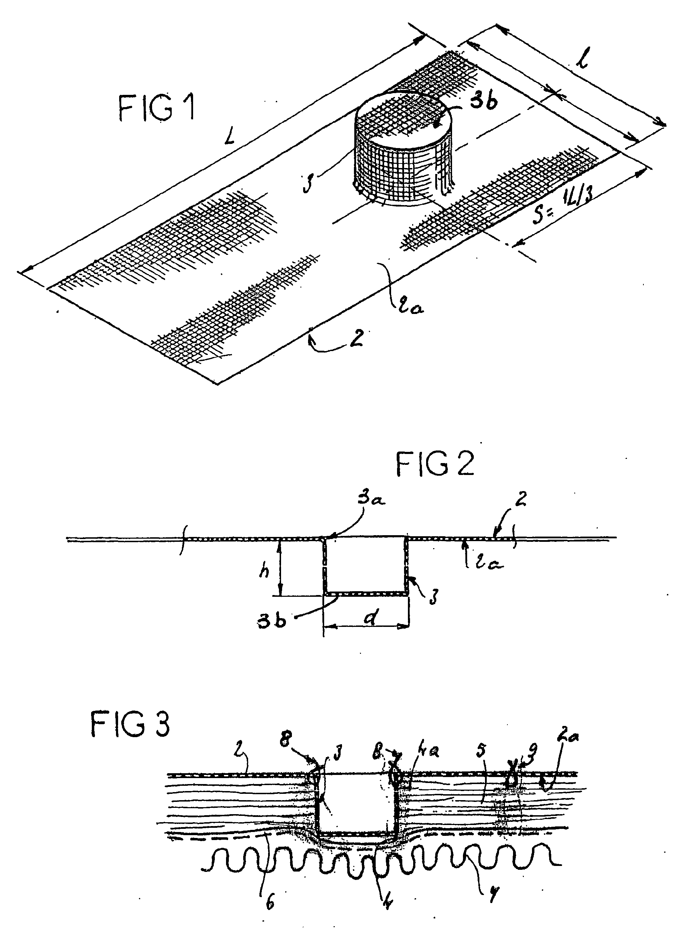

Dans la forme d'exécution montrée aux figures 1 et 2, l'implant est composé d'une partie plane 2, de forme générale rectangulaire, à angles droits ou arrondis. De l'une des faces de cette partie plane 2 fait saillie une excroissance 3 en forme de cuvette cylindrique dont l'axe longitudinal est perpendiculaire à la partie plane 2. L'ouverture 3a de la cuvette que forme l'excroissance est située dans le plan de la partie plane 2, et le fond 3b de cette excroissance 3, opposé à l'ouverture 3a, est plat et parallèle à la partie plane 2.

La partie plane 2 et l'excroissance 3 sont monolithiques et réalisées dans un tricot en monofilaments ou multifilaments de polypropylène ou polyéthylène ayant un diamètre compris entre 0,10 et 0,20 millimètre.

Selon l'invention, l'implant est réalisé à partir d'un tricot de monofilaments de polypropylène de 0,15 mm, à mailles ouvertes, ce tricot étant formé de deux nappes tricotées selon les barèmes respectifs suivants : nappe 1 : 32/01/12/43 ; nappe 2 : 32/01/12/43.

Ces armures forment des mailles indémaillables donnant au tricot une semi-rigidité avec néanmoins une possibilité de déformation.

L'excroissance cylindrique 3 est obtenue par thermoformage de ce tricot.

Selon une possibilité de mise en oeuvre, la partie plane 2 est comprimée entre deux plaques métalliques, de mêmes dimensions, dont l'une comporte un plot ayant les dimensions intérieures de l'excroissance devant être réalisée et dont l'autre comporte une ouverture ayant le diamètre extérieur de l'excroissance à réaliser. Les deux plaques sont chauffées à une température de l'ordre de 140 °C pendant environ 25 secondes. La chaleur et l'effort mécanique d'engagement du plot dans l'ouverture de l'autre plaque, associée à la possibilité de déformation du tricot constituant la partie plane, forment dans celle-ci une excroissance qui, en sortie de fabrication, présente une relative rigidité et a une paroi cylindrique, d'axe sensiblement perpendiculaire au plan de la partie plane.

La pièce ainsi formée est ensuite soumise à un temps de refroidissement par air de l'ordre de 15 secondes.

Le tricot n'est pas pincé fortement lors de l'emboutissage et peut donc glisser entre les plaques lors de l'aménagement de l'excroissance. Ce glissement permet de conserver une densité de matériau restant importante au niveau de l'excroissance.

Après emboutissage, la pièce formée présente les caractéristiques suivantes :
- densité du tricot
   au niveau de la partie plane : 84 g/m² environ ;
   au niveau du fond 3b : 72 g/m² environ ;
   au niveau de la paroi périphérique de l'excroissance 3 : 80 g/m² environ ;
- résistance à la rupture du tricot au niveau de la partie plane :
   sens longitudinal : 490 N
   sens transversal : 318 N
- résistance à la déchirure du tricot au niveau de la partie plane :
   sens longitudinal : 24 N
   sens transversal : 69N
- résistance en compression de l'excroissance :
   1.7 N pour une excroissance d'environ 25 mm de diamètre et 18 mm de hauteur
   2.6 N pour une excroissance une excroissance d'environ 25 mm de diamètre et de 14 mm de hauteur.

Selon une autre possibilité de mise en oeuvre, le tricot est disposé entre deux plaques métalliques chauffées à température réglable et comportant, en vis à vis l'une de l'autre, deux alésages cylindriques aptes à être traversés par un mandrin chauffant.

Dans une forme d'exécution préférée, la partie plane 2 est rectangulaire a une largeur l de l'ordre de 60 millimètres pour une longueur L de l'ordre de 120 millimètres, tandis que l'excroissance 3 a un diamètre d de l'ordre de 20 millimètres pour une hauteur h qui n'est pas supérieure à 20 millimètres et par exemple est de 15 millimètres.

L'excroissance 3 est aménagée de manière à être sensiblement au milieu de la largeur de la partie plane 2 et au tiers de sa longueur par rapport à l'une de ses extrémités, comme montré par la distance S sur la figure 1.

La face profonde 2a de la partie plane 2, c'est-à-dire la face de laquelle fait saillie l'excroissance 3, peut être rugueuse pour augmenter ses conditions d'accrochage et d'adhérence aux structures anatomiques postérieures. Cette rugosité résulte de la texture et/ou de l'armure utilisée.

L'implant est destiné à assurer une réparation herniaire par voie ouverte antérieure. Lorsqu'il est mis en place, et comme le montre la figure 3, l'excroissance 3 a des dimensions lui permettant de s'adapter à toutes les ouvertures herniaires 4 formées dans les muscles 5 juxtaposés au fascia transversalis 6 et au péritoine 7. La liaison de l'excroissance 3 avec les bords 4a de l'ouverture musculaire 4 est assurée par des sutures 8.

La partie plane 2, qui s'étend autour de l'ouverture 4 contre la paroi inguinale, renforce cette dernière en évitant les récidives. Cette partie plane 2 est fixée par quelques points de suture 9.

Lorsque l'implant est ainsi mis en place, l'excroissance 3 s'insère parfaitement dans l'ouverture musculaire 4, sans générer de réaction indiquant la présence d'un corps étranger. Son sommet plat et atromatique, ne dépassant pas de l'épaisseur de la paroi, limite les risques de lésions viscérales profondes par érosion. De plus, grâce à la rigidité de son matériau constitutif et de sa réalisation par thermoformage, l'excroissance 3 ne risque pas de se déformer ou de s'éverser.

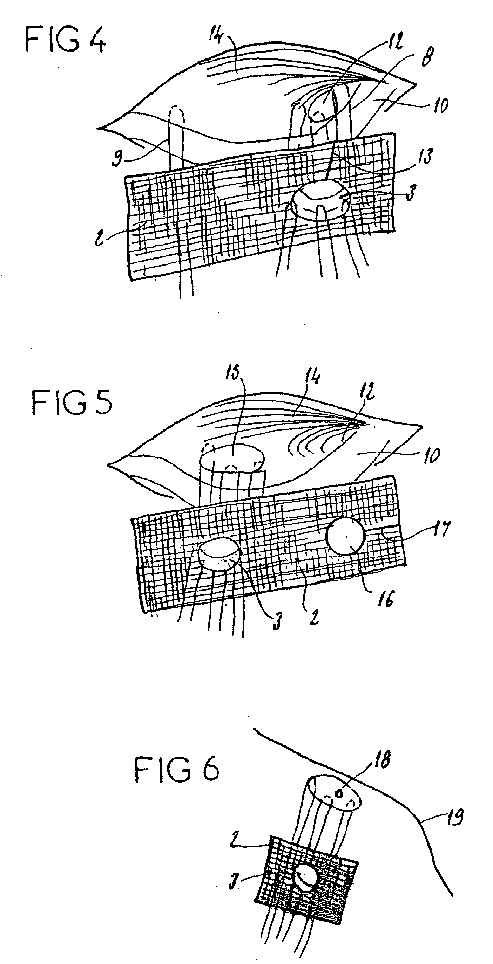

La figure 4 montre la mise en place d'un implant selon l'invention pour une hernie indirecte gauche. Pour faciliter l'engagement de l'excroissance 3 dans l'anneau inguinal 12 et permettre le passage du cordon spermatique 10, le chirurgien adapte l'implant en réalisant dans celui-ci une fente 13 permettant de laisser passer ce cordon 10. Sur le dessin, la référence 14 indique les muscles obliques externes et les références 8 et 9 indiquent les fils de suture décrits en référence à la figure 3.

Le chirurgien peut adapter la forme extérieure de la partie plane 2 à la taille de la dissection, afin d'épouser parfaitement celle-ci.

La figure 5 montre la mise en place d'un implant selon l'invention dans le cadre de la réparation d'une hernie directe gauche. Il s'agit ici de réparer un orifice 15 ménagé dans le triangle de Hesselbach, dans une zone plus éloignée de l'anneau inguinal 12. Pour cette réparation, l'implant est retourné de 180° par rapport à la position qu'il occupe à la figure 3, de manière qu'après la mise en place de l'excroissance 3 dans l'orifice 15, la plus grande partie de la partie plane 2 s'étende en direction de l'anneau inguinal 12. Pour permettre le passage du cordon spermatique 10, le chirurgien réalise, dans la partie plane 2, une ouverture 16 avec une fente 17 et, éventuellement, adapte la forme extérieure de la partie plane 2 à la forme de la dissection.

La figure 6 montre le traitement d'une hernie crurale droite, qui nécessite d'obturer l'anneau fémoral 18, disposé à proximité du ligament inguinal 19, au moyen de l'excroissance 3. Dans ce cas, le chirurgien adapte l'implant en le sectionnant en longueur de façon à lui donner sensiblement la même longueur de part et d'autre de l'excroissance 3.

Il ressort de ce qui précède que l'implant selon l'invention est universel puisqu'il peut s'appliquer à la restauration des hernies indirectes, directes et crurales et que, dans toutes ces applications, il assure non seulement l'obturation de l'orifice herniaire, mais également le renforcement du plancher inguinal autour de cet orifice, en réduisant les risques de récidive, et.. ce, sans pour autant former un corps dont la compacité pourrait être une source de gêne pour le patient.

## Revendications

1. Implant complet et universel pour la réparation des hernies par voie antérieure, composé d'une partie plane (2, 22) en matériau biocompatible, monofilaments, tricotée avec une armure lui conférant indémaillabilité et macroporosité, et d'une excroissance creuse (3, 23) en matériau biocompatible saillant d'une face de cette partie plane avec laquelle elle forme un ensemble monolithique ; la partie plane (2, 22) est plate et s'étend sur une surface lui permettant de couvrir la paroi postérieure du canal inguinal, et l'excroissance (3, 23) est disposée au milieu de la largeur de la partie plane et sensiblement au tiers de sa longueur, ladite excroissance ayant un axe longitudinal perpendiculaire à la partie plane (2, 22), formant une ouverture (3a) située dans le plan de la partie plane, et présentant un fond (3b), opposé à l'ouverture, plat et parallèle à la partie plane ;
l'excroissance (3, 23) étant en forme de cuvette cylindrique et ledit fond (3b), opposé à ladite ouverture (3b), étant espacé de celle-ci par une distance (h) dont la valeur n'est pas supérieure à la valeur du cliamètre (d) de la cuvette (3, 23), implant réalisé à partir d'un tricot de monofilaments de polypropylène de 0,15 mm, à mailles ouvertes, **caractérisé en ce que** ce tricot est formé de deux nappes tricotées selon les barèmes respectifs suivants : nappe 1 : 32/01/12/43 ; nappe 2 : 32/01/12/43.

2. Implant selon la revendication 1, **caractérisé en ce que** l'excroissance (3, 23) en forme de cuvette à un diamètre (d) dont la valeur est de l'ordre de 20 millimètres et une hauteur (h) dont la valeur n'est pas supérieure à cette dimension.

3. Implant selon la revendication 1, **caractérisé en ce que** la partie plane plate (2, 22), de forme générale rectangulaire à extrémités droites ou arrondies, a une largeur de l'ordre de 60 millimètres et une longueur de l'ordre de 120 millimètres, pour constituer autour de l'excroissance (3, 23), formant obturateur après son introduction dans l'ouverture (4, 15, 18) d'une paroi, un implant plat renforçant ladite paroi et pouvant être lié à elle par des sutures (9) éloignées de l'ouverture.

4. Implant selon la revendication 1, **caractérisé en ce que** la face (2a) de la partie plane (2, 22) qui est destinée à venir en contact avec les structures anatomiques postérieures présente une rugosité résultant de la texture et/ou de l'armure du tricot composant cette partie plane.

5. Implant selon la revendication 1, **caractérisé en ce que** l'excroissance en forme de cuvette (3) est réalisée par déformation à chaud de la partie plane (2), dont la texture et/ou l'armure lui donnent une semi-rigidité.

6. Implant selon la revendication 1, **caractérisé en ce que** sa partie plane (2, 22) et son excroissance (3, 23) sont imprégnées par des substances bactéricides et anti-inflammatoires ou analgésiques.

7. Implant selon la revendication 1, **caractérisé en ce que** sa partie plane (2, 22) présente localement une fente (13) pour le passage du cordon spermatique.

8. Procédé d'obtention de l'implant selon l'une des revendications 1 à 7, **caractérisé en ce qu'**il comprend les étapes consistant à :
- comprimer le tricot entre deux plaques métalliques, dont l'une comporte un plot ayant les dimensions intérieures de l'excroissance devant être réalisée ou est apte à être traversée par un tel plot, et dont l'autre comporte une ouverture ayant le diamètre extérieur de l'excroissance ;
- chauffer les deux plaques et le plot ;
- engager ledit plot dans l'ouverture de l'autre plaque de manière à former dans le tricot une excroissance rigide et présentant une paroi sensiblement cylindrique, sensiblement à angle droit par rapport à la surface plane du tricot,
le rapprochement des plaques étant tel que le tricot peut glisser entre les plaques lors de l'aménagement de l'excroissance au moyen dudit plot.

## Claims

1. A complete and universal implant for the repair of hernias by an anterior approach, composed of a plane part(2, 22) of biocompatible material, made of monofilaments, knitted with a structure which prevents unraveling and provides macroporosity, and of a hollow protuberance (3, 23) made of biocompatible material protruding from one face of this plane part with which it forms a one-piece assembly; the plane part(2, 22) is flat and extends over a surface allowing it to cover the posterior wall of the inguinal canal, and the protuberance (3, 23) is situated at the middle of the width of the plane part and approximately one third of the way along its length, said protuberance having a longitudinal axis perpendicular to the plane part (2, 22), forming an opening (3a) situated in the plane of the plane part, and having a base (3b) which lies opposite the opening and is flat and parallel to the plane part;
the protuberance (3, 23) being in the form of a cylindrical cell, and said base (3b), lying opposite said opening (3b), being spaced apart from the latter by a distance (h) whose value is not greater than the value of the diameter (d) of the cell (3, 23), the implant being made from a knit of 0.15 mm polypropylene monofilaments, with open meshes, **characterized in that** this knit is formed of two knitted sheets in accordance with the following respective configurations: sheet 1: 32/01/12/43; sheet 2: 32/01/12/43.

2. The implant as claimed in claim 1, **characterized in that** the protuberance (3, 23) in the form of a cell has a diameter (d) whose value is of the order of 20 millimeters and a height (h) whose value is not greater than that dimension.

3. The implant as claimed in claim 1, **characterized in that** the flat plane part(2, 22), of overall rectangular shape with straight or rounded ends, has a width of the order of 60 millimeters and a length of the order of 120 millimeters, so as to form, around the protuberance (3, 23), forming an obturator after its introduction into the opening (4, 15, 18) of a wall, a flat implant which strengthens said wall and is able to be connected to it by sutures (9) arranged away from the opening.

4. The implant as claimed in claim 1, **characterized in that** the face (2a) of the plane part (2, 22) intended to come into contact with the posterior anatomical structures has a roughness resulting from the texture and/or the structure of the knit from which this plane part is composed.

5. The implant as claimed in claim 1, **characterized in that** the protuberance in the shape of a cell (3) is produced by thermal deformation of the plane part(2) whose texture and/or structure give it semi-rigidity.

6. The implant as claimed in claim 1, **characterized in that** its plane part (2, 22) and its protuberance (3, 23) are impregnated by substances which are bactericidal and anti-inflammatory or analgesic.

7. The implant as claimed in claim 1, **characterized in that** its plane part(2, 22) has, locally, a slit (13) for the passage of the spermatic cord.

8. A method for obtaining the implant as claimed in one of claims 1 through 7, **characterized in that** it comprises the steps of:
- compressing the knit between two metal plates, one of which comprises a plug having the internal dimensions of the protuberance to be formed or is capable of being engaged by such a plug, and the other of which comprises an opening having the external diameter of the protuberance;
- heating the two plates and the plug;
- engaging said plug in the opening of the other plate in such a way as to form, in the knit, a rigid protuberance with a substantially cylindrical wall which is substantially at right angles to the flat surface of the knit, the movement of the two plates toward each other being such that the knit can slide between the plates during formation of the protuberance by means of said plug.

## Patentansprüche

1. Vollständiges und universelles Implantat für die Hernienreparation auf vorderem Wege, das aufgebaut ist aus einem ebenen Teil (2, 22) aus biokompatiblem rnononfilamentigem Material, der mit einer Armierung gewirkt ist, die ihm eine Maschenfestigkeit und eine Makroporosität verleiht, und aus einer hohlen Ausstülpung (3, 23) aus biokompatiblem Material, die von einer Seite des ebenen Teils vorspringt, mit dem sie eine einstückige Gesamtheit bildet; der ebene Teil (2, 22) ist flach und erstreckt sich über eine Fläche, die eine Bedeckung der hinteren Wand des Leistenkanals ermöglicht, und die Ausstülpung (3, 23) ist in der Mitte der Breite des ebenen Teils und etwa im Drittel seiner Länge angeordnet, wobei die Ausstülpung, die eine Längsachse senkrecht zu dem ebenen Teil (2, 22) aufweist, eine Öffnung (3a) bildet, die sich in der Ebene des ebenen Teils befindet, und einen Boden (3b) aufweist, der der Öffnung entgegengesetzt angeordnet, flach und parallel zu dem ebenen Teil ist;
wobei die Ausstülpung (3, 23) in Form eines zylindrischen Topfes ausgebildet ist, und der Boden (3b), der der Öffnung (3b) entgegengesetzt ist, von dieser um einen Abstand (h) beabstandet ist, dessen Betrag nicht größer ist als der Betrag des Durchmessers (d) des Topfes (3, 23), wobei das Implantat ausgehend von einem Gewirke aus Monofilamenten aus Polypropylen von 0,15 mm mit offenen Maschen gefertigt ist, **dadurch gekennzeichnet, dass** dieses Gewirke aus zwei Lagen gebildet ist, die gemäß den jeweiligen folgenden Schemata gewirkt sind: Lage 1: 32/01/12/43; Lage 2: 32/01/12/43.

2. Implantat nach Anspruch 1, **dadurch gekennzeichnet, dass** die Ausstülpung (3, 23) in Form des Topfes einen Durchmesser (d), dessen Betrag in der Größenordnung von 20 mm liegt und eine Höhe (h) aufweist, dessen Betrag nicht größer als diese Abmessung ist.

3. Implantat nach Anspruch 1, **dadurch gekennzeichnet, dass** der flache ebene Teil (2, 22) von allgemein rechteckiger Form mit geraden oder abgerundeten Enden eine Breite in der Größenordnung von 60 mm und eine Länge in der Größenordnung von 120 mm aufweist, um um die Ausstülpung (3, 23) herum, die nach ihrer Einführung in die Öffnung (4, 15, 18) einer Wand einen Obturator bildet, ein flaches Implantat zu bilden, das diese Wand verstärkt und mit dieser durch von der Öffnung beabstandete Nähte (9) verbunden werden kann.

4. Implantat nach Anspruch 1, **dadurch gekennzeichnet, dass** die Seite (2a) des ebenen Teils (2, 22), die dazu bestimmt ist, mit den hinteren anatomischen Strukturen in Berührung zu kommen, eine Rauheit aufweist, die aus der Textur und/oder der Armierung des Gewirkes, aus dem dieser ebene Teil aufgebaut ist, resultiert.

5. Implantat nach Anspruch 1, **dadurch gekennzeichnet, dass** die Ausstülpung in Form des Topfes (3) durch Warmumformung des ebenen Teils (2) gefertigt ist, dessen Textur und/oder Armierung ihm eine Halbsteifigkeit verleihen.

6. Implantat nach Anspruch 1, **dadurch gekennzeichnet, dass** sein ebener Teil (2, 22) und seine Ausstülpung (3, 23) mit Bakteriziden und entzündungshemmenden oder analgetischen Substanzen imprägniert sind.

7. Implantat nach Anspruch 1, **dadurch gekennzeichnet, dass** sein ebener Teil (2, 22) lokal einen Schlitz (13) für den Durchgang des Samenleiters aufweist.

8. Verfahren zur Herstellung des Implantats nach einem der Ansprüche 1 bis 7, **dadurch gekennzeichnet, dass** es die folgenden Schritte aufweist:
- Einspannen des Gewirkes zwischen zwei Metallplatten, von denen die eine ein Formungsteil aufweist, das die Innenabmessungen der zu fertigenden Ausstülpung aufweist, oder geeignet ist, von einem solchen Formungsteil durchquert zu werden, und von denen die andere eine Öffnung aufweist, die den Außendurchmesser der Ausstülpung aufweist;
- Erwärmen der beiden Platten und des Formungsteils;
- In-Eingriff-Bringen des Formungsteils mit der Öffnung der anderen Platte derart, dass in dem Gewirke eine steife Ausstülpung gebildet wird, die eine etwa zylindrische, bezüglich der ebenen Fläche des Gewirkes etwa senkrechte Wand aufweist,
wobei das Aufeinanderzubewegen der Platten derart erfolgt, dass das Gewirke während der Ausbildung der Ausstülpung mittels des Formungsteils zwischen den Platten gleiten kann.
